(19) Europäisches Patentamt

European Patent Office

Office européen des brevets

(11) EP 0 530 689 B1

(12) **EUROPÄISCHE PATENTSCHRIFT**

(45) Veröffentlichungstag und Bekanntmachung des Hinweises auf die Patenterteilung:
**13.01.1999 Patentblatt 1999/02**

(51) Int. Cl.[6]: **A61M 1/36**

(21) Anmeldenummer: **92114688.2**

(22) Anmeldetag: **28.08.1992**

(54) **Vorrichtung zur Rezirkulation im Single-Needle-Verfahren bei der Zellseparation**

Device for recirculation in a single needle process for the separation of blood cells

Appareil de recirculation dans un procédé à aiguille unique pour la séparation de cellules sanguines

(84) Benannte Vertragsstaaten:
**DE ES FR GB IT**

(30) Priorität: **06.09.1991 DE 4129639**

(43) Veröffentlichungstag der Anmeldung:
**10.03.1993 Patentblatt 1993/10**

(73) Patentinhaber: **Fresenius AG**
**61350 Bad Homburg (DE)**

(72) Erfinder:
**Neumann, Hans-Jürgen, Dr.**
**D-6690 St. Wendel-Niederkirchen (DE)**

(74) Vertreter:
**Luderschmidt, Schüler & Partner GbR**
**Patentanwälte,**
**John-F.-Kennedy-Strasse 4**
**65189 Wiesbaden (DE)**

(56) Entgegenhaltungen:
**EP-A- 0 132 210**     **EP-A- 0 155 684**
**EP-A- 0 392 304**     **WO-A-88/02641**
**DE-A- 3 931 471**     **DE-C- 4 129 271**

## Beschreibung

Die Erfindung betrifft eine Vorrichtung zur Trennung von Blut nach dem Oberbegriff des Patentanspruchs 1.

Eine Vorrichtung der eingangs erwähnten Art ist beispielsweise aus der DE 39 31 471 A1 oder dem Journal of Clinical Apheresis, Band 6, Seiten 24-27 (1991) bekannt.

Das Einnadelverfahren bietet gegenüber den kontinuierlichen Zweinadelverfahren den Vorteil, daß der Spender nur ein einziges Mal mit einer Kanüle punktiert werden muß. Dies ist insbesondere von Vorteil, wenn schlechte Punktierungsverhältnisse vorliegen.

Andererseits sind bei dem Einnadelverfahren zwei Zyklen durchzuführen, nämlich ein Kollektionszyklus, bei dem das von einer Vene abgezogene Blut zur Separation einer Zentrifuge zugeführt wird, in der die zu sammelnden und zurückzuführenden Blutfraktionen getrennt werden, und ein Rückgabezyklus, bei dem die zurückzuführenden Fraktionen zum Spender zurückgefördert werden, beispielsweise mittels einer zweiten Pumpe, wie dies in der vorgenannten Offenlegungsschrift beschrieben ist oder mittels einer auf einen Speicherbeutel Druck ausübenden Einrichtung.

Bei dem Einnadelverfahren treten zwei Probleme auf, nämlich eine vom Spender abhängende Toleranz bezüglich des dem Blut zugemischten Antikoagulans und die teilweise eingeschränkte Effektivität der Separation.

Was zunächst die Toleranz gegen das Antikoagulans betrifft, das zumeist in Form von ACD-A zugegeben wird, so wird letzteres von den Spendern bei der Rückgabe in die Armvene unterschiedlich gut vertragen. Bei hohen Rückgabeflüssen des Bluts gelangen ebenso hohe Anteile Antikoagulans zum Spender zurück. Andererseits verlängert sich bei kleinen Rückgabeflüssen die Rückgabezeit in entsprechender Weise. Wünschenswert ist daher eine Möglichkeit zur Einstellung des Rückgabeflusses.

Was nun die teilweise beschränkte Effektivität der Separation betrifft, so läßt sich diese - wie dies bereits in der deutschen Offenlegungsschrift genannt ist - durch eine Rezirkulation des zurückgegebenen Bluts verbessern. In der Offenlegungsschrift ist jedoch nicht unmittelbar angegeben, mit welchen Steuerungsmitteln bei der dort beschriebenen Blutpumpe als Zuführungspumpe und einer in die Rückgabeleitung eingeschalteten zweiten Pumpe das geschehen soll.

Der Erfindung liegt daher die Aufgabe zugrunde, die Vorrichtung der eingangs erwähnten Art derart zu verbessern, daß einerseits eine vorbestimmte Blutmenge dem Spender zur Verbesserung der Toleranz zurückgeführt werden kann und andererseits die Effektivität der Separation gesteigert werden kann.

Diese Aufgabe wird durch die kennzeichnenden Merkmale des Anspruchs 1 gelöst.

Was zunächst die erfindungsgemäße Vorrichtung betrifft, so weist diese nur eine einzige Pumpe in Form einer Blutpumpe im Zuführungszweig auf, während im Abführungszweig lediglich eine auf den Speicherbeutel Druck ausübende Druckeinrichtung vorgesehen ist, gegen deren Druck der Speicherbeutel in der Kollektionsphase gefüllt wird und die auf den Speicherbeutel in der Rückgabephase vorteilhafterweise einen gleichbleibenden Druck ausübt. Eine derartige Druckeinrichtung ist in der mit dem Titel "Vorrichtung zum Entleeren von flexiblen Flüssigkeitsbehältern" eingereichten deutschen Patentanmeldung mit der Publikationsnummer DE-A-4129271 beschrieben, auf deren Offenbarung Bezug genommen wird.

Demzufolge wird also in der Rückgabephase nicht eine mit einer vorbestimmten Förderphase arbeitende aktive Rückführpumpe, sondern vielmehr eine passive Druckeinrichtung eingesetzt, so daß der in dem gesamten Rückführsystem vorliegende Gesamtflußwiderstand ausschlaggebend ist für die dem Spender zurückgeführte Blutmenge.

Dieses Rückführsystem besteht üblicherweise aus drei Komponenten, nämlich dem Beutel einschließlich einer Verbindungsleitung zur Rückführleitung, der Rückführleitung selbst bis zum Y-Stück und der vom Y-Stück abgehenden Leitung einschließlich der Nadel selbst.

Im einzelnen besteht der Gesamtflußwiderstand also aus den Teilflußwiderständen $R_P$ für den Innenwiderstand des Beutels einschließlich Zuführleitung, $R_0$ für den Zuführschlauchwiderstand und $R_N$ für den Nadelwiderstand. Sämtliche Teilflußwiderstände summieren sich zum Gesamtflußwiderstand $R_{GES}$.

Erfindungsgemäß beträgt der Flußwiderstand der Rückführleitung das 0,2 - 0,8fache, insbesondere etwa die Hälfte des Gesamtwiderstands $R_{GES}$, wobei 'etwa' eine Abweichung von ± 20 % bedeutet. Mit einem solchen Verhältnis läßt sich ein Nadelrückfluß $F_N$ in medizinisch sinnvollen Grenzen von 50 - 80 ml/min einstellen, wobei zugleich eine Rezirkulationsrate $F_R$ im technisch für die Zentrifuge sinnvollen Bereich von 20 - 80 ml/min einstellbar ist. Zusätzlich ist in einem solchen Fall ein Druck $P_0$ mittels der Beutelpresse in einem Bereich von etwa $1,331 \cdot 10^4$ Pa (100 mmHg) vorzugeben.

Diese Parameter ergeben folgende Geradengleichung:

$$F_N = l/R_{GES} \cdot (P_0 - R_0 \cdot F_R).$$

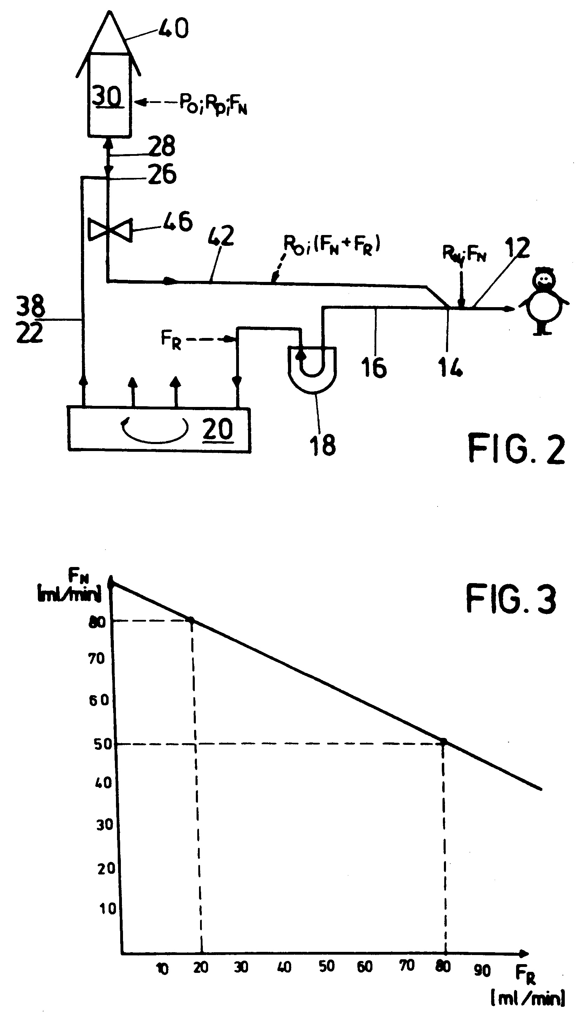

In Abbildung 3 ist die Gerade für die Flüsse $F_N$ und $F_R$ dargestellt, wobei die Steigung der Geraden sich aus dem Verhältnis von $R_0 / R_{GES}$ ergibt, das wie angegeben, einen Wert von etwa 0,5 einnimmt.

Aus der vorstehenden Geradengleichung läßt sich ohne weiteres $R_0$ bzw. das Verhältnis von $R_0 / R_{GES}$ bestimmten. Zum einen ist die Flußrate IR der Blutpumpe vorgebbar. Zum anderen kann mit Hilfe von bekannten Druckflußmessern der Nadelrückfluß $F_N$ unmittelbar bestimmt werden. Ist der von der Druckpresse ausgeübte Druck konstant, so kann direkt die Steigung der Geraden aus Abbildung 3 ermittelt werden, d.h. das Verhältnis $R_0 / R_{GES}$ kann in bestimmten Grenzen noch eingestellt werden. So kann $R_0$ dadurch erhöht werden, daß in der Rückführleitung eine einstellbare Klemme aktiviert wird, die den Flußwiderstand erhöht oder erniedrigt. Desgleichen kann natürlich auch ein vorgewähltes Schlauchsystem mit einem bestimmten Innendurchmesser und einer bestimmten Länge, d.h. einem vorbestimmten Durchflußwiderstand , eingesetzt werden. Schließlich kann natürlich auch der Durchflußwiderstand des Rests des Rückgabesystems, insbesondere der Nadelwiderstand, durch Wahl unterschiedlicher Nadelgrößen eingestellt werden. Üblicherweise wird jedoch der Durchflußwiderstand des Rückführschlauchs entweder mittels einer einstellbaren Klemme verändert oder aber bereits vorgewählt eingesetzt. So läßt sich beispielsweise ein für die Blutzellseparation einsetzbarer Schlauch mit einem Innendurchmesser von etwa 3 mm und den dort eingesetzten Längen und Schlauchsegmenten vorteilhaft einsetzen.

Betrachtet man nunmehr die in Abbildung 3 angegebenen Verhältnisse, so ist ersichtlich, daß ohne Rezirkulation ($F_R = 0$) ein Reinfusionsfluß von 90 ml/min erreicht werden kann, was nur durch die Einstellung des Gesamtwiderstands zu erreichen ist.

Andererseits können durch Aktivierung der Blutpumpe als Rezirkulationspumpe unterschiedliche Reinfusionsflüsse, wie dies eingangs gefordert worden ist, erzielt werden, beispielsweise bei einem Rezirkulationsfluß von 80 ml/min ein Reinfusionsfluß von 50 ml/min. Dieser Reinfusionsfluß dauert natürlich nur so lange, bis der Speicherbeutel geleert ist, woraufhin ein neuer Kollektionszyklus eingeleitet wird.

Es sei im übrigen noch darauf verwiesen, daß der Rückfluß nicht nur durch den Druck auf den Speicherbeutel bestimmt ist, sondern vielmehr auch abhängig ist vom Hämatokritwert und von der Viskosität des Bluts, wobei letztere Parameter während der Behandlung weitgehend als Konstanten zu betrachten sind.

Die Menge des tolerierbaren Antikoagulans-Flusses durch die Rückflußleitung hängt von der Größe, dem Gewicht und dem Geschlecht des Spenders ab. So verträgt eine kleine leichte Spenderin wesentlich weniger Antikoagulans pro Zeiteinheit als ein großer schwerer Spender. Für diese Zusammenhänge kann eine Referenztabelle benutzt werden, wobei zusätzlich der Hämatokrit und die Viskosität berücksichtigt werden können. Eine solche Tabelle wird in der Steuervorrichtung der erfindungsgemäßen Vorrichtung vorteilhafterweise abgelegt. Eine solche Einstellung kann durch einen Mikroprozessor des Zellseparators selbst durchgeführt werden, indem diese Tabelle und die spenderspezifischen Werte eingegeben werden können. Der Mikroprozessor wählt also in Abhängigkeit vom Hämatokrit, Größe, Gewicht, Geschlecht des Spenders einen Rezirkulationsfluß aus, bei dem der Rückfluß in den Spender, die Reinfusion, eine Sicherheitsgrnze nicht überschreitet. Dies wird im wesentlichen mit den eingangs gegebenen Flußraten erreicht.

Die mit der erfindungsgemäßen Vorrichtung durchgeführte Rezirkulation bei dem Einnadel- - Verfahren führt zu einer weiteren und somit verbesserten Separation der abzutrennenden Blutbestandteile, insbesondere der Thrombozyten. Werden beispielsweise in einer 5-minütigen Kollektionsphase etwas 50 % der Thrombozyten des prozessierten Bluts in einem Kollektionsbeutel gesammelt, können in der Rückgabephase bei 5-minütiger Rezirkulation weitere 50 % der verbliebenen Thrombozyten abgeschöpft werden, so daß insgesamt 75 % aller abtrennbaren Thrombozyten nunmehr separiert worden sind.

Die Erhöhung der Effektivität ist um so höher, je höher der Rezirkulationsfluß und je länger die Reinfusion dauert, wobei diese Zeitspannen gegeneinander laufen.

Wie bereits vorstehend erwähnt, ist der Rückfluß bei konstanter Rezirkulationsrate durch die Veränderung des Flußwiderstandes der Rückführleitung einstellbar, beispielsweise durch Verwendung einer handelsüblichen Rollenklemme.

In Abhängigkeit von der Verträglichkeit des Antikoagulans für den Spender läßt sich durch die Wahl der Kanüle weiterhin der maximale Rückgabefluß bestimmen. Bei gleichem Rezirkulationsfluß wird bei einer dünnen Kanüle weniger zum Spender zurückfließen als bei einer dickeren. Die ermittelten Werte lassen sich für verschiedene Kanülen und Rückgabeflüsse dabei in einem Diagramm ablesen.

Im folgenden wird die Erfindung anhand eines Ausführungsbeispieles näher erläuert.

Es zeigen

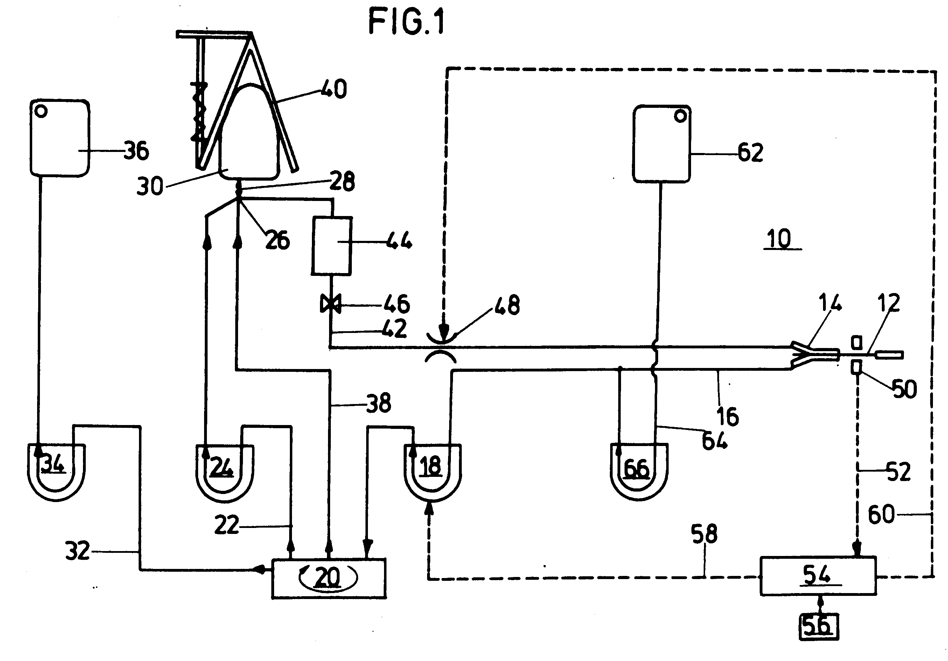

Figur 1      eine schematische Anordnung einer erfindungsgemäßen Vorrichtung.

Eigur 2      eine weiter schematisierte Anordnung der Vorrichtung gemäß Figur 1 und

Figur 3      eine grafische Darstellung, in der die Abhängigkeit des Nadelrückflusses vom Rezirkulationsfluß bei dem Einsatz der erfindungsgemäßen Vorrichtung gezeigt ist.

In Figur 1 ist mit 10 eine Ausführungsform der erfindungsgemäßen Vorrichtung gezeigt. Diese Vorrichtung 10 weist eine Kanüle 12 zum Anschluß an einen Patienten auf, deren anderes Ende mit einem Y-Stück 14 verbunden ist. Vom Y-Stück geht eine Zuführungsleitung 16 ab, in die eine Blutpumpe 18 eingeschaltet ist. Das Ende der Zuführungsleitung 16 ist mit einer Zentrifuge oder Separationseinrichtung 20 verbunden, in der eine nicht gezeigte Separationskammer angeordnet ist.

Von der Zentrifuge geht eine Plasmaleitung 22 ab, in die eine Plasmapumpe 24 eingeschaltet ist. Das Ende der Plasmaleitung 22 ist über ein Verzweigungsstück 26 und eine Verbindungsleitung 28 mit einem Speicherbeutel 30 verbunden.

Des weiteren geht von der Zentrifuge 20 eine Thrombozytenleitung 32 ab, in die eine Thrombozytenpumpe 34 eingeschaltet ist und an deren Ende ein Thrombozytenaufnahmebeutel 36 angeordnet ist.

Schließlich geht von der Zentrifuge 20 eine Erythrozytenleitung 38 ab, die ebenfalls über das Verzeigungsstück 26 und die Verbindungsleitung 28 mit dem Speicherbeutel 30 verbunden ist.

Der Speicherbeutel 30 selbst ist innerhalb einer Druckpresse 40 angeordnet, die auf den Beutel einen vorbestimmten Druck ausübt, wobei dieser Druck im wesentlichen vom Entleerungsgrad des Speicherbeutels 30 unabhängig ist.

Vom Verzweigungsstück 26 geht eine Rückführungsleitung 42 ab, deren Ende mit dem Y-Stück 14 verbunden ist. In diese Rückführleitung 42 ist im Beispielsfalle eine Troptkammer 44 und eine Klemme 46 sowie eine Drossel 48 eingeschaltet, mit der der Querschnitt der Rückführleitung 42 veränderbar ist.

Schließlich ist im Bereich der Kanüle 14 vorteilhafterweise eine Durchflußmeßanordnung 50 vorgesehen, die ihr Signal über die Leitung 52 an ein Rezirkulationssteuergerät 54 abgibt. Dieses Rezirkulationssteuergerät 54 ist mit einer Eingabe 56 verbunden und steuert selbst über die Leitungen 58 und 60 die Blutpumpe 18 in der Rezirkulationsphase und gegebenenfalls die Drossel 48.

Aus Übersichtlichkeitsgründen ist ein weiteres Steuergerät, mit dem die Vorrichtung zwischen den beiden Separations- und Rückgabezyklen umgeschaltet wird, nicht gezeigt.

In Figur 2 sind die Bezugszeichen gemäß Figur 1 für die gleichen Elemente verwandt worden. Zusätzlich wurden hier die Parameter für die einzelnen Elemente eingesetzt.

Dabei bedeuten

$R_N$ = Nadelwiderstand,
$R_0$ = Rückflußleitungswiderstand,
$R_P$ = Innenwiderstand Beutelpresse und Verbindungsleitung,
$F_R$ = Rezirkulationsfluß,
$F_N$ = Nadelfluß,
$P_0$ = Druck-Beutelpresse.

Die Parameter hängen über die eingangs genannte Gleichung voneineinander ab.

Die Vorrichtung gemäß Figur 1 wird folgendermaßen betrieben.

Es wird ein Schlauchsystem ausgewählt, bei dem $R_0$ etwa die Hälfte des Gesamtwiderstandes bestehend aus $R_N$, $R_0$ und $R_P$ ist. Gegebenenfalls wird durch Messung mit dem Durchflußmeßgerät und Verstellung der Drossel 48 nach Auswertung durch die Rezirkulationssteuereinrichtung 54 die Drossel 48 verstellt (entweder der Schlauchdurchmesser erweitert oder verengt).

Über die Eingabe 56 können patientenspezifische Daten sowie die angestrebten Nadel- und Rezirkulationsflüsse eingegeben werden. Der im Steuergerät 54 befindliche Rechner kann dann über den vorstehend genannten Algorithmus die Rezirkulationsgeschwindigkeit der Blutpumpe 18 einstellen und gegebenenfalls die Drossel 48 zur Veränderung des Widerstands der Rückführleitung 42 verändern.

Der Betrieb der Vorrichtung 10 selbst erfolgt wie er beispielsweise in der eingangs genannten deutschen Offenlegungsschrift beschrieben ist, worauf Bezug genommen wird. Insofern wird in der Kollektionsphase zunächst die Blutpumpe 18 aktiviert, wobei die Klemme 46 geschlossen bleibt. Zugleich wird die Pumpe 66 zur Zuführung von Antikoagulans in Betrieb genommen. Die einzelnen in der Zentrifuge 20 getrennten Blutbestandteile werden mit Hilfe der Plasmapumpe 24 bzw. der Thrombozytenpumpe 34 abgeführt, wobei zusätzlich die Blutpumpe 18 die Erythrozyten in den Speicherbeutel 30 befördert, in dem sie mit dem Plasma wieder vermischt werden.

Nach Beendigung der Kollektionsphase erfolgt die Rückführphase mit dem Rezirkulationsschritt. Hierzu wird die Klemme 46 geöffnet und die Blutpumpe 18 wird mit der Förderrate für die Rezirkulation betrieben. Da das Blut bereits mit Antikoagulans versehen ist, wird die Antikoagulanspumpe 66 angehalten, die über die Leitung 64 von einem Antikoagulansbeutel 62 das Antikoagulans der Zuführleitung 16 zufördert. Andererseits werden die Plasmapumpe 24 und die Thrombozytenpumpe 34 weiterbetrieben.

Nach Beendigung des Rückführzyklus und der Rezirkulation wird erneut in den Separationszyklus umgeschaltet, in dem das Blut des Patienten der Zentrifuge 20 zugeführt wird.

Mit Hilfe der erfindungsgemäßen Vorrichtung 10 kann also durch die Rezirkulation weniger Koagulans je Zeiteinheit dem Patienten zurückgeführt und darüber hinaus die Effektivität der Separation erhöht werden.

**Patentansprüche**

1. Vorrichtung zur Trennung von Blut mit einer Kanüle (12), einem mit der Kanüle verbundenen Y-Stück (14), einer vom Y-Stück (14) abgehenden Zuführungsleitung (16), in die eine Blutpumpe (18) eingeschaltet ist, eine mit der Zuführungsleitung (16) verbundenen Zentrifuge (20) mit Separationskammer, einer von der Separationskammer abgehenden und zur Rückführung in den Speicherbeutel (30) mündende Leitung (22), in die eine Plasmapumpe (24) eingeschaltet ist, einer von der Separationskammer abgehenden und zum Sammeln in einen Kollektionsbeutel (36) mündende Leitung (32), einer Rückführleitung (42), die mit dem Speicherbeutel (30) und am Y-Stück (14) mit der Kanüle (12) verbunden ist, einem Antikoagulans enthaltenden Antikoagulans-Beutel (62), der über eine Nebenleitung (64), in die eine Antikoagulans-Pumpe (66) eingeschaltet ist, mit der Zuführungsleitung (16) verbunden ist, einer Pumpeinrichtung (40) zum Rückführen des gespeicherten Blut-Plasmagemisches aus dem Speicherbeutel (30) sowie mit einem Steuergerät zum wechselweisen Aktivieren der Blutpumpe (18) und Antikoagulans-Pumpe (66) einerseits und des Rückführsystems andererseits, indem es von einer Kollektionsphase in eine Rückgabephase und zurück schaltet,
dadurch gekennzeichnet,
daß die Pumpeinrichtung (40) eine auf den Speicherbeutel (30) Druck ausübende, vorspannbare Druckeinrichtung (40) ist und daß der Flußwiderstand $R_0$ der Rückführleitung (42) das 0,2-0,8fache des Flußwiderstandes $R_{GES}$ des gesamten Rückführsystems beträgt, das aus dem Speicherbeutel (30) einschließlich einer Verbindungsleitung zur Rückführleitung (42), der Rückführleitung (42) selbst bis zum Y-Stück (14) und der vom Y-Stück (14) abgehenden Leitung einschließlich der Kanüle (12) besteht und daß Steuermittel (54) für die Blutpumpe (18) vorgesehen sind zur Einstellung eines vorbestimmten Rezirkulationsflusses durch die Leitung (16) zur Zentrifuge (20).

2. Vorrichtung nach Anspruch 1, dadurch gekennzeichnet, daß der Flußwiderstand $R_0$ der Rückführleitung (42) die Hälfte des Flußwiderstandes $R_{GES}$ des gesamten Rückfuhrsystems beträgt, mit einer Toleranz von ± 20% dieses Wertes.

3. Vorrichtung nach Anspruch 1 oder 2, dadurch gekennzeichnet, daß sie Flußwiderstände ($R_B$, $R_0$, $R_N$) im Rückführsystem aufweist, welche bei einem vorgegebenen Druck $P_0$ der Druckeinrichtung (40) von 1,331 $10^4$ Pa (100 mm Hg) einen Nadelfluß $F_N$ von 50 bzw. 80 ml/min bei einem vorbestimmten Rezirkulationsfluß $F_R$ von 80 bzw. 20 ml/min bewirken, wobei die Rezirkulationsförderraten $F_R$ mit den Nadelflußraten $F_N$ entsprechend der Gleichung

$$F_N = 1/R_{GES} \times (P_0 - R_0 \times F_R)$$

verknüpft sind.

4. Vorrichtung nach einem der Ansprüche 1 bis 3, dadurch gekennzeichnet, daß in der Rückführleitung (42) eine verstellbare Drossel (48) angeordnet ist, mit der der Flußwiderstand $R_0$ der Rückführleitung (42) einstellbar ist.

5. Vorrichtung nach Anspruch 4, dadurch gekennzeichnet, daß im Bereich der Kanüle (12) ein Durchflußmesser (50) angeordnet ist, dessen Signal von einem Rezirkulationssteuergerät (54) zur Aktivierung der Drossel (48) und/oder der Rezirkulationspumpe (18) eingesetzt wird.

6. Vorrichtung nach Anspruch 5, dadurch gekennzeichnet, daß das Rezirkulationssteuergerät (54) mit einer Eingabeeinheit (56) verbunden ist, mit der patientenspezifische Daten sowie Flußraten von Nadel (12) und der Blutpumpe (18) eingebbar sind.

## Claims

1. An apparatus for the separation of blood, with a cannula (12), Y-piece (14) connected to the cannula, a feed line (16) extending from the Y-piece (14) and into which a blood pump (18)is connected, a centrifuge (20) connected to the feed line (16) and with a separating chamber, a line (22) emerging from the separating chamber and, for recycling, opening into the storage bag (30) and with an incorporated plasma pump (24), a line (32) extending from the separating chamber and, for collection, emerging into a collection bag (36), a return line (42) which is connected to the storage bag (30) and on the Y-piece (14) with the cannula (12), an anticoagulant bag (42) containing an anticoagulant and which is connected to the feed line (16) via a secondary line (64) into which an anticoagulant pump (66) is incorporated, a pump means (40) for returning the stored blood-plasma mixture from the storage bag (30) and with a control means for alternately activating the blood pump (18) and the anticoagulant pump (66) on the one hand and the recycling system on the other, in that it switches from a collection phase to a return phase and back, characterised in that the pump means (40) is a pretensionable pressure means (40) which exerts a pressure on the storage bag (30) and in that the flow resistant $R_0$ of the return line (42) amounts to 0.2 to 0.8 times the flow resistance $R_{GES}$ which consists of the storage bag (30) including a connecting line to the return line (42), the return line (42) itself as far as the Y-piece (14) and the line emerging from the Y-piece (14) including the cannula (12) and in that control means (54) for the blood pump (18) are provided for adjusting a predetermined recirculating flow through the line (16) to the centrifuge (20).

2. Apparatus according to claim 1, characterised in that the flow resistance $R_0$ of the return line (42) amounts to half the flow resistance $R_{GES}$ of the entire recycling system with a tolerance of ± 20% of this value.

3. Apparatus according to claim 1 or 2, characterised in that in the recycling system it has flow resistances ($R_B$ $R_0$, $R_N$) which for a given pressure $P_0$ in the pressure means (40) of 1.331 $10^4$ Pa (100 mm Hg) produces a needle flow $F_N$ of 50 and/or 80 ml/min for a predetermined recirculation flow $F_R$ of 80 and/or 20 ml/min, the recirculation delivery rates $F_R$ being linked to the needle flow rates $F_N$ according to the equation

$$F_N = 1/R_{GES} \times (P_O - R_O \times F_R).$$

4. An apparatus according to one of claims 1 to 3, characterised in that there is in the return line (42) an adjustable choke (48) with which it is possible to adjust the flow resistance $R_O$ in the return line (42).

5. Apparatus according to claim 4, characterised in that there is in the region of the cannula (12) a flow meter (50) the signal from which is used by a recirculation control unit (54) to activate the choke (48) and/or the recirculating pump (18).

6. Apparatus according to claim 5, characterised in that the recirculation control unit (54) is connected to an input unit (56) with which it is possible to feed in patient-specific data as well as flow rates of needle (12) and blood pump (18).

## Revendications

1. Dispositif de séparation du sang comprenant une canule (12), une pièce en Y (14) reliée à la canule, une ligne d'alimentation (16) partant de la pièce en Y (14) dans laquelle est disposée une pompe à sang (18), une centrifugeuse (20) reliée à la ligne d'alimentation (16) et possédant une chambre de séparation, une ligne (22) partant de la chambre de séparation et débouchant dans la poche de stockage (30) pour le retour dans laquelle est disposée une pompe à plasma (24), une ligne (32) partant de la chambre de séparation et débouchant dans une poche de collecte (36) pour le recueil, une ligne de retour (42) reliée à la poche de stockage (30) et par l'intermédiaire de la pièce en Y (14) à la canule (12), une poche d'anticoagulant (62) reliée à la ligne d'alimentation (16) par l'intermédiaire d'une ligne auxiliaire (64) dans laquelle est disposée une pompe à anticoagulant (66), un dispositif de pompe (40) pour recycler le mélange sang/plasma à partir de la poche de stockage (30) ainsi qu'un appareil de commande pour activer alternativement d'une part la pompe à sang (18) et la pompe à anticoagulant (66) et d'autre part le système de recyclage en passant d'une phase de collecte à une phase de restitution et inversement, caractérisé en ce que le dispositif de pompe (40) est un dispositif de pression (40) pouvant être précontraint et exerçant une pression sur la poche de stockage (30) et en ce que la résistance à l'écoulement $R_0$ de la ligne de retour (42) est égale à 0,2 à 0,8 fois la résistance à l'écoulement $R_{TOT}$ de l'ensemble du système de recyclage composé de la ligne de raccordement à la ligne de retour (42), de la ligne de retour (42) proprement dite jusqu'à la pièce en Y (14) et de la ligne partant de la pièce en Y, (14) y compris la canule (12), et en ce qu'il est prévu des moyens de commande (54) pour la pompe à sang (18) afin de fixer un débit de recirculation prédéterminé dans la ligne (16) vers la centrifugeuse (20).

2. Dispositif selon la revendication 1, caractérisé en ce que la résistance à l'écoulement $R_0$ de la ligne de retour (42) est égale à la moitié de la résistance à l'écoulement $R_{TOT}$ de l'ensemble du système de recyclage, avec une tolérance de ± 20 % par rapport à cette valeur.

3. Dispositif selon la revendication 1 ou la revendication 2, caractérisé en ce qu'il présente des résistances à l'écoulement ($R_P$ $R_0$, $R_N$) dans le système de recyclage qui pour une pression $P_0$ donnée du dispositif de pression (40) égale à 1,331 · $10^4$ Pa (100 mm Hg) génèrent un débit de l'aiguille $F_N$ de 50 ou 80 ml/min pour un débit de recirculation $F_R$ prédéterminé de 80 ou 20 ml/min, les débits de recirculation $F_R$ étant liés aux débits de l'aiguille $F_N$ par l'équation :

$$F_N = 1/R_{TOT} \times (P_0 - R_0 \times F_R).$$

4. Dispositif selon l'une quelconque des revendications 1 à 3, caractérisé en ce qu'un clapet d'étranglement (48) réglable permettant de régler la résistance à l'écoulement $R_0$ de la ligne de retour (42) est disposé dans la ligne de retour (42) .

5. Dispositif selon la revendication 4, caractérisé en ce qu'un capteur de mesure de débit (50) dont le signal est utilisé par un appareil de commande de recirculation (54) pour activer le clapet d'étranglement (48) et/ou la pompe de recirculation (18) est disposé dans la zone de la canule (12).

6. Dispositif selon la revendication 5, caractérisé en ce que l'appareil de commande de recirculation (54) est relié à une unité de saisie (56) permettant d'entrer des données spécifiques au patient ainsi que des débits pour l'aiguille (12) et la pompe à sang (18) .

FIG.1

FIG. 2

FIG. 3